# EUROPEAN PATENT SPECIFICATION

(11) **EP 0 373 962 B1**
(45) Date of publication and mention of the grant of the patent: **13.09.1995**
(21) Application number: 89313157.3
(22) Date of filing: 15.12.1989
(51) Int. Cl.: C12N 15/52, C12N 9/00, C12Q 1/25

(54) **Isolating thermostable enzymes**
Gewinnung von thermostabilen Enzymen
Isolation d'enzymes thermostables

(30) Priority: 16.12.1988 US 285128
(43) Date of publication of application: 20.06.1990
(73) Proprietor: ABBOTT LABORATORIES, Abbott Park, Illinois 60064-3500 (US)
(72) Inventor: Backman, Keith C., Bedford Massachusetts 01730 (US); Rudd, Edwin A., Salem New Hampshire 03079 (US); Lauer, Gail, Cambridge Massachusetts 02138 (US); McKay, Diane, Arlington Massachusetts 02174 (US)
(74) Representative: Rackham, Anthony Charles

(56) References cited:
- EP-A- 258 017
- WO-A-89/06691
- CHEMICAL ABSTRACTS, vol. 94, no. 5, 02.02.1981, Columbus, Ohio, USA YANG, Q.-S. et al.
- JOURNAL OF BIOLOGICAL CHEMISTRY, vol. 259, no. 16, 25.08.1984, BALTIMORE US, TAKAHASHI, M. ET AL.

## Description

This invention relates to the field of producing and recovering thermostable enzymes, particularly enzymes having activity related to nucleic acid metabolism (e.g. RNA or DNA polymerases, ligases, endonucleases or exonucleases).

There are various uses for thermostable enzymes having nucleic acid metabolic activity.

Our European Patent Application No. 88311741.8 (published under No. EP-A-0320308), the disclosure of which is to be regarded as hereby incorporated by reference, discloses an assay in which DNA ligase is used in repetitive cycles of first hybridizing probe pairs to a target, then ligating the hybridized probes, and finally denaturing the hybridized ligated probes. By such cycles, the ligated probes are amplified and detected, so as to provide an amplified signal representative of the target. In this assay, it is desirable to use a thermostable DNA ligase, e.g. from Thermus thermophilus, which can withstand the high temperature required for denaturing the DNA without substantial loss of ligase activity, so that the next ligation cycle is accomplished without adding additional ligase.

U.S. Patents 4,683,195 and 4,683,202 describe a method of amplifying target DNA in a sample, using primers, triphosphates, and DNA polymerase. EP application 0258017 discloses isolation of thermostable DNA polymerase from Thermus aquaticus for use in that amplification procedure. The use of a thermostable enzyme in the procedure is said to avoid the need to add additional enzyme after each denaturation step. EP '017 also reports cloning a Thermus aquaticus DNA polymerase gene and expressing it in E. coli strain DG116 (ATCC 53606).

Takahashi et al. (1984) J. Biol. Chem. 259:10041-10047, describe the purification and properties of the thermostable DNA ligase from Thermus thermophilus. Other references, Romaniec et al. (1987) J. Gen. Microbiol. 133:1297-1308, Liao et al. (1986) Proc. Natl. Acad. Sci. USA 83:576-580, Hara-Yokoyama et al. (1984) J. Biochem. 96:1599-1608, Matsumara et al. (1985) J. Biol. Chem. 260:15298-15303 describe the purification and properties of various thermostable enzymes.

This invention has arisen in the course of our work seeking to provide a straightforward way to separate thermostable enzymes from the contaminants that accompany their in vivo production. When separating the thermostable enzyme from its natural background, troublesome thermostable contaminants are difficult to avoid. For example, when purifying a thermostable ligase or polymerase, it may be difficult to avoid proteases which may be detrimental to the amplification procedures described above. Other thermostable DNA metabolizing enzymes, such as DNA endonucleases also may seriously undermine the effectiveness of the above-described amplification assays. Even if the gene encoding the thermostable enzyme is cloned into a non-thermostable background, the host will produce its own DNA metabolizing enzymes, and proteases. Thus, cloning by itself, only trades one set of contaminants for another.

As will become clear from the detailed description which follows, our techniques provide an opportunity to effectively denature and remove host organism proteins, quickly and easily, possibly in as little as a single step with minimal risk to the desired thermostable enzyme. This enables an abundant, and relatively low-cost source of thermostable enzymes, without detrimental contaminants that otherwise may be difficult to remove.

In accordance with a first aspect of the present invention, there is provided a method for obtaining a thermostable ligase enzyme essentially free from unwanted contaminants, characterised in comprising the steps of:
(a) providing a mesophilic host cell engineered to express a gene encoding a heterologous thermostable ligase enzyme, the gene being characterised in that it codes for a protein having substantially the same thermostable ligating activity as the protein encoded by the gene found in the approximately 2.6kb Bst XI - Bg1 II fragment found in pGL628;
(b) culturing the mesophilic host cell to produce the thermostable enzyme in a mixture comprising unwanted contaminants; and
(c) purifying the thermostable enzyme, that purification comprising at least one step in which a mixture comprising the unwanted contaminants is heated to a temperature sufficient to inactivate the unwanted contaminants but not sufficient to inactivate the thermostable enzyme.

In this context, purifying the thermostable enzyme means reducing the abundance or activity level of contaminating substances (e.g. endonucleases, exonucleases, proteases) particularly those which interfere with the intended use of the enzyme such as in the selective amplification procedure described above, or with recovery of the enzyme. Mesophilic host cells are cells which can be engineered to produce the desired thermophilic enzyme and whose proteins generally are denatured at a temperature that does not denature the desired thermophilic enzyme. Representative suitable mesophilic host cells are described below. Heterologous, as used above, means that the enzyme is not naturally produced by the host cell.

In preferred embodiments, the mesophilic host cells are lysed after culturing, to produce a lysate comprising the thermostable enzyme and unwanted contaminating proteins. Preferably, the heating step essentially completely denatures and precipitates the undesired contaminants, so that the contaminants are readily separated from liquid phase containing the desired thermostable enzyme. Particularly preferred enzymes are DNA ligases .

Preferred enzymes are those from thermophilic bacteria (described in more detail below) which survive a heating step of 65 _{°} C to about 100 _{°} C (most preferably 80 °C-95 °C), for a sufficient period of time (e.g. at least about 1-3 minutes, and preferably for at least 5 minutes) to inactivate mesophilic host cell proteins.

Our described techniques overcome cloning- related difficulties experienced in obtaining thermostable ligases, such as the difficulty of easily assaying for ligase activity when screening transformants, and the difficulty of obtaining high levels of ligase expression in transformants.

In particular, a second aspect of the invention features a method of assaying for NAD-dependent nucleic acid ligase activity (particularly thermostable ligase activity) by forming a stable covalent adenyl (e.g. from labeled NAD) adduct with the ligase, and detecting the adduct. Ligase activity present in a sample comprising undesired contaminants can be detected by heating the sample under conditions which denature the contaminants. This is particularly useful where any contaminants which would also form adenyl adducts are denatured.

This assay method may be used to screen host cells transformed with a library of nucleic acid segments, thereby identifying cells which express ligase activity.

A third aspect of the invention generally features an isolated nucleic acid gene, e.g. nucleic acid encoding a protein having nucleic acid (DNA) ligase activity, which encodes a thermostable ligase enzyme, the gene being characterised in that it codes for a protein having substantially the same thermostable ligating activity as the protein encoded by the gene found in the approximately 2.6kb Bst XI - Bg1 II fragment found in pGL628, wherein the nucleic acid is part of an engineered cloning vehicle in which that gene does not naturally occur.

A fourth aspect of the invention features an essentially pure DNA sequence encoding a thermostable nucleic acid ligase enzyme, the sequence being characterised in that it codes for a protein having substantially the same thermostable ligating activity as the protein encoded by the gene found in the approximately 2.6kb Bst XI - Bg1 II fragment found in pGL628.

Preferably, in the third and fourth aspects, the cloned gene is part of an expression vehicle (a plasmid or other transformable DNA segment) comprising a promoter that provides enhanced (e.g. at least twice the level of gene product in unengineered cells) expression of the gene.

Other features and advantages will be apparent from the following description.

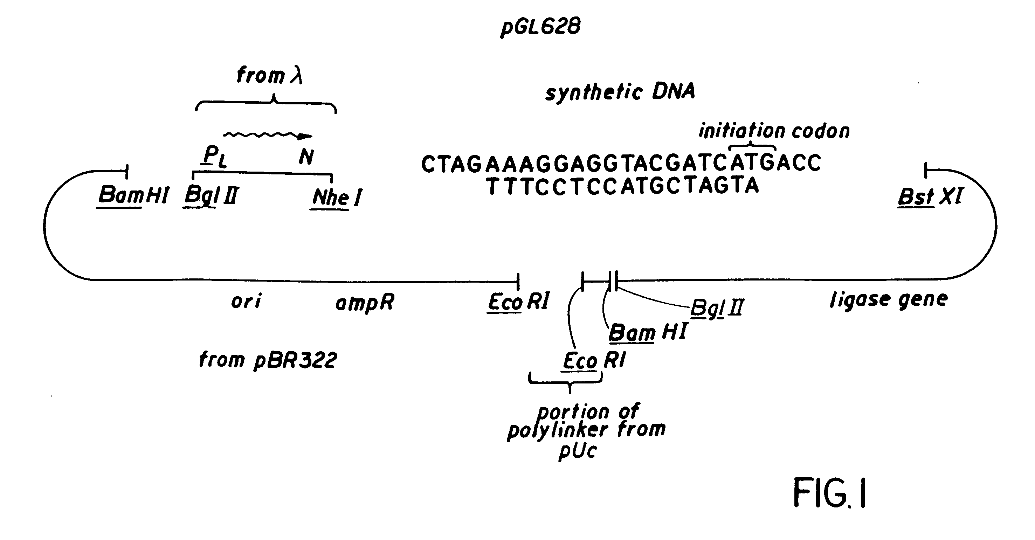

Fig. 1 is a diagrammatic representation of the components of plasmid pGL628 that contains a Thermus thermophilus DNA fragment extending from a BstXl site to a Bg111 site about 2.6 kb away.

Those skilled in the field will recognize that there are numerous ways to perform the various steps in the method for a wide variety of enzymes. Generally, the techniques of recombinant DNA manipulation, expression and cell culturing are well-known techniques understood in the art. The mesophilic host cells used in the method can be procaryotes represented by various strains of E. coli. However, other microbial strains may also be used, such as bacilli, for example Bacillus subtilis, various species of Pseudomonas, or other bacterial strains. In such procaryotic systems, plasmid vectors that contain replication sites and control sequences derived from the host or from a species compatible with the host are used. In addition to bacteria, eucaryotic microbes, such as yeast, may also be used as hosts. Laboratory strains of Sac- charomyces cerevisiae, Bakers' yeast, are most often used, although a number of other strains are commonly available.

It is also of course, possible to express genes encoding the thermophilic enzymes in eucaryotic host cell cultures derived from multicellular organisms. See, for example, Tissue Culture, Academic Press, Cruz and Patterson, editors (1973). Useful host cell lines include murine myelomas N51, VERO, MD canine kidney cells, HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al., Nature (1978) 273:113), or other viral promoters such as those derived from polyoma, Adenovirus 2, bovine papilloma virus, or avian sarcoma viruses, or immunoglobulin promoters and heat shock promoters. A system for expressing DNA in mammalian systems using the BPV as a vector is disclosed in U.S. Patent 4,419,446. A modification of this system is described in U.S. Patent 4,601,978. General aspects of mammalian cell host system transformations have been described by Axel, U.S. Patent No. 4,399,216. Origins of replication may be obtained, if needed, from viral sources. However, integration into the chromosome is a common mechanism for DNA replication in eucaryotes.

Plant cells are also now available as hosts, and control sequences compatible with plant cells such as the nopaline synthase promoter and polyadenylation signal sequences (Depicker, A., et al., J.Mol.Appl.Gen. (1982) 1:561) are available.

Recently, in addition, expression systems employing insect cells utilizing the control systems provided by baculovirus vectors have been described (Miller, D.W., et al., in Genetic Engineering (1986) Setlow, J.K. et al., eds., Plenum Publishing, Vol. 8, pp. 277-297).

Construction of suitable vectors containing the desired coding and control sequences employs standard ligation and restriction techniques that are well understood in the art. Isolated plasmids, DNA sequences, or synthesized oligonucleotides are cleaved, tailored, and religated in the form desired.

Site-specific DNA cleavage is performed by treating with the suitable restriction enzyme (or enzymes) under conditions that are generally understood in the art, and the particulars of which are specified by the manufacturer of these commercially available restriction enzymes. See, e.g., New England Biolabs, Product Catalog.

Cloning of genes encoding thermostable nucleic acid metabolic genes, e.g. T. thermophilius DNA ligase, is significantly assisted by development of an effective method for detecting the activity of such enzymes. In particular, where the enzymes are expressed only at extremely low levels in the cloning background, detection may be so difficult that traditional strategies predicated on identifying clones which express the desired gene (e.g. complementation of deficient mutants or immunological identification of desired transformants) are unlikely to be effective without such an improved screening method.

The problem is exacerbated by lack of characterization of the desired protein, which hampers cloning strategies based on sequence data. In particular, T. thermophilus ligase has not been sequenced because of poor yields of enzyme and impurities (particularly proteolytic impurities).

Accordingly, Example 1 regarding a method for detecting the nucleic acid metabolic enzyme (particularly thermostable DNA ligase) is an important aspect of our cloning strategy. Other Examples are provided concerning cloning and expression. These Examples are provided to illustrate the invention, but not to limit it.

### Example 1 - Detection of the Thermophilic Enzyme

The following observations provide a method of detecting a thermophilic enzyme which can be labeled with NAD, e.g. Thermus thermophilus ligase, in a mixture of host proteins.

NAD is most often utilized as a mediator of oxidation/reduction reactions, and very few enzymes utilize NAD as a source of stored chemical energy. Prokaryotic DNA ligases are one class of enzyme which does. Most other energy consuming enzymatic processes, including certain DNA ligases, utilize ATP as an energy source. Very few prokaryotic enzymes survive and remain active at temperatures in excess of 70 °C. Aside from idiosyncratic examples, the majority of such enzymes exist in thermophilic organisms.

It is known that some nucleic acid metabolic enzymes (e.g. DNA ligase) form a covalently linked enzyme adenylate species as an intermediate in the reaction sequence they catalyze. We have discovered that at temperatures between 70 °C and 80 °C, thermostable DNA ligase from the thermophile Thermus thermophilus can form a stable, covalent adenyl adduct, and that the adduct can be specifically labelled with ^{32p} if the NAD used as a cofactor is labelled with ^{32p} (available from New England Nuclear). The enzyme has an extremely low K_{M} for NAD, so the above reaction is satisfactory for labelling the cloned ligase product, even though extremely small quantities of thermophilic ligase are expressed in E. coli prior to engineering for increased expression.

In general, mesophilic host proteins either cannot be labeled at all with NAD, or their ability to be labeled is substantially eliminated by previous exposure to 70 _{°} C or higher.

In outline, extracts suspected of containing thermophilic ligase are incubated at 70 _{°} C or higher; ³²P-NAD is added; samples are incubated further; samples are denatured and run on SDS polyacrylamide gel electrophoresis; the gel is autoradiographed; and the presence or absence of DNA ligase in a sample is ascertained by the presence or absence of a labelled species which comigrates with authentic ligase.

More specifically, cells are grown and cell extracts are assayed as follows.

The cells from a plate containing 70 colonies transformed with a library of cloned DNA segments from T. thermophilus are scraped into 3.0 ml of L broth. This suspension was used to inoculate 100 ml of L broth in a 2 liter flask. The cells were grown at 37° C until they had reached saturation (- 3-4 hrs.). The cells from a 20 ml aliquot of the culture were harvested by centrifugation, and the cell pellet was stored at -20 _{°} C.

The cell pellets described above were suspended in 10 ml of disruption buffer (20mM TRIS, 0.1 mM EDTA and 1 mM β-mercaptoethanol, pH 7.6). The cells were disrupted by sonication. The cell debris was removed by centrifugation (20 min. at 20,000 rpm, 4_{°}C, SS-34 rotor). The resulting supernatant was incubated in a 80° C water bath for 30 minutes. The precipitated proteins were then removed by centrifugation (20 min., 7,000 rpm at 4° C, SS-34 rotor). The resulting supernatant was placed in an Amicon Centriprep™ (10,000 MWCO) and concentrated to = 0.5 ml. The concentrate was transferred to an Amicon Centricon™ (10,000 MWCO) and the process of concentration was continued until a volume of = 50 µl was reached. The concentrated samples were then placed in plastic microfuge tubes and incubated in an 80° C water bath for 20 minutes. Precipitate was again removed by centrifugation (4 min. in microfuge) and the supernatant was transferred to another plastic microfuge tube. The tube was again placed in the 80 ° C water bath After the sample had reached temperature, 1.0 µl of diluted ³²P-NAD was added and the tube was incubated for 2 hours at 80 ° C (NEN stock ³²P-NAD was diluted 1/2 in 0.2M TRIS, pH 7.6). Then 25 µl of Laemmli SDS gel sample buffer was added to the tube and the incubation at 80° C was continued for another 20 minutes. The sample was then electrophoresed on a 10% Laemmli SDS-gel. When the tracking dye neared the bottom of the gel the electrophoresis was stopped, and the bottom of the gel containing most of the ^{32p} label (NAD) was cut away. The gel was soaked in H₂0 for 30 minutes and then dried on a gel dryer. The dried gel was then autoradiographed. The antoradiograph was analyzed for the presence of species which comigrated with authentic ligase during electrophoresis.

### Example 2 - Assay of DNA Ligase

Ligase can be semi-quantitatively assayed by a nick sealing procedure. Plasmid DNA (Puc19) is nicked by treatment with BamHl endonuclease in the presence of ethidium bromide. (pUC18 and many other plasmids could also be used.) Ligase is assayed by treatment of a fixed amount of nicked plasmid with dilutions of samples containing ligase, followed by agarose gel electrophoresis in the presence of ethidium bromide. Conversion of nicked to CCC form DNA is assessed and correlated with dilution. The assay is especially useful for determining thermophilic ligase activity when the reaction is run at a temperature between 50 ° C and 75 _{°} C.

The specific DNA ligase assay is conducted in the following buffer: 50mM Tris, 10mM MgC1₂, 50mM KCI, 2mM MnC1₂, 1mM DTT, 10µg/ml BSA, and 0.1 mM NAD. Ligation is performed using a mixture that is 2µl DNA ligase containing sample, 1µl DNA (1µg/ml nicked DNA), and 7µl of the above buffer. The mixture is incubated for 30 min. at 68 _{°} C and ligation is stopped using 6µl of 50mM EDTA/0.5% SDS, and 0.05% BPB.

To detect ligase, 5µl of the 16µl mix is electrophoresed on a gel of 0.8% agarose with 1.0µl/ml ethidium bromide, TBE running buffer. A positive control is run using E. coli DNA ligase.

### Example 3 - Cloning of Thermophilic Ligase

T. thermophilus cells (ATCC 27634) were grown and DNA was prepared from gently lysed cells by equilibrium density gradient centrifugation in CsCI. DNA was partially digested with Sau3AI and cloned in the Bcll site of the positive selection vector pTR264, a version of pTR262 (Roberts et al., Gene 12:123 (1980)) which contains an ampicillin resistance gene. Tetracycline resistant clones (i.e. those having inserts) were isolated. Pools of 70 colonies were made, and lysates of such pools were examined for the presence of thermophilic ligase by the procedure of Example 1. A positive pool was identified, and broken into sub-pools of 7 colonies. By repeating the procedure and breaking down the subpool to single colonies, a clone containing thermophilic ligase was identified. It carried a plasmid which was named pGL600.

The identity of the NAD-labelling species was confirmed to be DNA ligase by assaying as described in Example 2.

### Example 4 - Location of DNA Ligase Gene

PGL600 contained about 8kb of inserted DNA. By subcloning, we localized the gene to a region of about 4kb (the coding capacity required to specify the gene was estimated to be about 2.2 kb). Since the subcloning was done in pUC18 and pUC19, identical subclones were obtained which were positioned oppositely with respect to an external lac promoter. Quantitation of the levels of ligase produced by such pairs of clones strongly suggested the orientation of the ligase gene.

Deletion of a Bglll bounded fragment near one end eliminated ligase formation and suggested that the beginning of the gene lay within that fragment.

The Bglll generated fragment was sequenced, and a putative start point for the ligase gene was identifed by analysis of the sequence.

### Example 5 - Expression of Thermophilic Ligase in E. coli

Although thermophilic ligase was detectably expressed in E. coli, the low and variable levels suggested to us that considerable improvement could be obtained. Analysis of the sequence revealed three useful facts.
1. The putative start codon is cleaved by the restriction endonuclease BstXl (See Fig. 1); further analysis revealed that this site was unique within the DNA spanning the gene;
2. The gene lacked an optimal ribosome binding site for expression in E. coli; and,
3. The gene apparently lacked an associated promoter conforming to the consensus promoter sequence in E. coli.

An appreciation of these observations informed the following procedure:
A DNA fragment comprising almost all of the gene was prepared from the BstXl site at one end to a Bglll site past the distal end of the gene. Synthetic DNA was prepared which could link the prepared DNA to a strong promoter; this synthetic DNA restored the initiator codon and provided a good ribosome binding site (See Fig. 1).

Several such constructions were made, varying the nature of the promoter and the ribosome binding site. For example the lac promoter from the pUC plasmid was used, the λ P_{L} promoter was used, and the M13 gene II promoter was used. DNA ligase production was monitored by polyacrylamide gel electrophoresis of cell lysates and by the assay procedure of Example 2.

One suitable construction resulting from the above procedure is a plasmid designated pGL628. Its components are shown in Fig. 1.

Construction of pGL628 was accomplished in cells which provided cl repressor to control expression from the P_{L} promoter on the plasmid U.S. patent application SN 091,837, filed September 1, 1987 by Keith C. Backman and Barbara Bolten, describes a method for preparing such strains. One such strain is KB649.

Plasmid pGL628 contains the -2.6 kb BstXl to Bgl II Thermus thermophilus DNA ligase DNA fragment. That plasmid, in E. coli, strain KB649 has been deposited in the American Type Culture Collection, Rockville, MD and given ATCC Accession No. 67858.

### Example 6 - Production of Ligase

Strain KB649/pGL628 was grown in a fermentor at 30° C in 10 lit res of minimal salts (e.g. M9 media) plus 15-20g/1 glucose. When the cells reached mid log phase, the temperature set point was changed to 42 °C, and incubation was continued for about 2 hours. Cells were harvested, and a cell paste was stored frozen. By examining cell lysates resolved by polyacrylamide gel elecrophoresis in the presence of sodium dodecyl sulfate, it was determined that between 5% and 10% of the total cell protein was thermophilic ligase.

### Example 7 - Ligase Purification from pGL628

Cell pellets (33g) were resuspended at 4ml/g in disruption buffer. Disruption buffer consisted of the following: 50mM Tris-HCI, 10mM MgC1₂, 50mM KCI, 2mM MnC1₂, 1mM DTT and 10mM NH₄CI, pH7.7

The disruption was carried out in a French Pressure cell at 14,000 PSI (9.65 x 10⁷ N/m²). The suspension was then spun down at 20K rpm for 20 minutes. The supernatant was removed and incubated in an 80° C H₂0 bath for 30 minutes. This suspension was then spun at 7.5K rpm for 15 minutes. The supernatant was then made up to 2% streptomycin sulfate using a 10% stock solution. This 2% solution was stirred on ice for 20 minutes, then spun at 12.5K rpm for 20 minutes.

This supernatant was loaded on a Cibacron blue column (136ml bed volume) equilibrated with 20mM Tris, 50 mM KCI, pH7.6. The column was eluted with a three-column volume gradient of 50 to 500 mM KCI. Fractions were collected in a 5 ml volume at a rate of 2.0 ml/min.

Active fractions were pooled, and subjected to an Amicon stirred cell concentrator, then an Amicon centriprep concentrator until the volume was 1-5% of the following gel filtration column. Specifically the sample was loaded on a Fractogel 50S column (bed volume 100 ml) equilibrated with 10mM KPO₄, 100mM KCI, 1mM MgC1₂, pH7.6, 5ml fractions were collected, at a ra̅t̅e̅ of 0.5ml/min.

Active fractions were pooled, concentrated in an Amicon Centriprep, made to 20% glycerol and stored at -20 ° C.

Other embodiments are feasible.

For example, other thermostable enzymes such as polymerases can be prepared.

## Claims (Claims for the following Contracting State(s) : AT, BE, CH, LI, DE, FR, GB, IT, LU, NL, SE)

1. A method for obtaining a thermostable ligase enzyme essentially free from unwanted contaminants, characterised in comprising the steps of:
(a) providing a mesophilic host cell engineered to express a gene encoding a heterologous thermostable ligase enzyme, the gene being characterised in that it codes for a protein having substantially the same thermostable ligating activity as the protein encoded by the gene found in the approximately 2.6kb Bst XI - Bg1 II fragment found in pGL628 (ATCC No.67858)
(b) culturing the mesophilic host cell to produce the thermostable enzyme in a mixture comprising unwanted contaminants; and
(c) purifying the thermostable enzyme, that purification comprising at least one step in which a mixture comprising the unwanted contaminants is heated to a temperature sufficient to inactivate the unwanted contaminants but not sufficient to inactivate the thermostable enzyme.

2. A method according to Claim 1, further characterised in that after said culturing step, the mesophilic host cell is lysed to produce a lysate comprising said thermostable enzyme.

3. A method according to Claims 1 or 2, further characterised in that said purification comprises heating said mixture comprising said unwanted contaminants to effect essentially completely precipitation of said unwanted contaminants from a liquid phase comprising the thermostable enzyme.

4. A method according to Claim 3, further characterised in that said unwanted contaminants comprise proteins, and said heating step denatures said proteins causing them to precipitate.

5. A method according to any preceding claim, further characterised in that said unwanted contaminants are endonucleases, exonucleases, or proteases.

6. A method according to any preceding claim, further characterised in that the thermostable enzyme is a DNA ligase.

7. A method according to Claim 6, further characterised in that the thermostable enzyme is a DNA ligase from a thermophilic bacteria.

8. A method according to Claim 7, further characterised in that the thermostable enzyme is from a thermophilic bacterium selected from Thermus flarus, Thermus ruber, Thermus thermophilus, Bacillus stearothermophilus, Thermus aquaticus, Thermus lacteus, Thermus rubens and Methanothermus fervidus.

9. A method according to any preceding claim, further characterised in that said temperature is from 65 _{°} C to about 100°C, and preferably from 80 _{°} C to about 95 _{°} C.

10. A method for assaying for NAD-dependent thermostable nucleic acid ligase activity, characterised in comprising forming a stable covalent AMP adduct of said ligase and detecting said adduct.

11. A method according to Claim 10, further characterised in that the adenyl moiety of said adduct is labelled.

12. A method according to Claims 10 or 11, further characterised in that the adenyl moiety of said adduct is derived from NAD.

13. A method according to any of Claims 10, 11 or 12, further characterised in that said ligase is a thermophilic ligase, and said ligase activity is detected in a sample comprising undesired contaminants; and in that said method comprises heating a mixture comprising said ligase and said undesired contaminants to a temperature which denatures or inactivates said undesired contaminants but not said ligase.

14. A method of cloning nucleic acid encoding nucleic acid ligase activity, in which a library of nucleic acid segments is transformed into host cells, characterised in that said transformed host cells are screened to identify cells which express ligase activity by a method according to any of Claims 10 to 13.

15. An isolated nucleic acid gene which encodes a thermostable ligase enzyme, the gene being characterised in that it codes for a protein having substantially the same thermostable ligating activity as the protein encoded by the gene found in the approximately 2.6kb Bst XI - Bg1 II fragment found in pGL628 (ATCC No. 67858)

16. A gene according to Claim 15, further characterised in that said gene is part of an expression vehicle comprising a promoter providing enhanced expression of said gene.

17. A gene according to claim 16, further characterised in that the expression vehicle is illustrated by the restriction map of Figure 1.

18. A gene according to Claim 16, further characterised in that the expression vehicle is that deposited under ATCC Accession NO: 67858 in E. Coli.

19. A gene according to any of Claims 15 to 18, further characterised in that the ligase is NAD-dependent ligase.

20. An essentially pure nucleic acid sequence which encodes a thermostable nucleic acid ligase enzyme, the sequence being characterised in that it codes for a protein having substantially the same thermostable ligating activity as the protein encoded by the gene found in the approximately 2.6kb Bst XI - Bg1 II fragment found in pGL628 (ATCC No. 67858)

## Claims (Claims for the following Contracting State(s) : ES, GR)

1. A method for obtaining a thermostable ligase enzyme essentially free from unwanted contaminants, characterised in comprising the steps of:
(a) providing a mesophilic host cell engineered to express a gene encoding a heterologous thermostable ligase enzyme, the gene being characterised in that it codes for a protein having substantially the same thermostable ligating activity as the protein encoded by the gene found in the approximately 2.6kb Bst XI - Bg1 II fragment found in pGL628 (ATCC No.67858)
(b) culturing the mesophilic host cell to produce the thermostable enzyme in a mixture comprising unwanted contaminants; and
(c) purifying the thermostable enzyme, that purification comprising at least one step in which a mixture comprising the unwanted contaminants is heated to a temperature sufficient to inactivate the unwanted contaminants but not sufficient to inactivate the thermostable enzyme.

2. A method according to Claim 1, further characterised in that after said culturing step, the mesophilic host cell is lysed to produce a lysate comprising said thermostable enzyme.

3. A method according to Claims 1 or 2, further characterised in that said purification comprises heating said mixture comprising said unwanted contaminants to effect essentially completely precipitation of said unwanted contaminants from a liquid phase comprising the thermostable enzyme.

4. A method according to Claim 3, further characterised in that said unwanted contaminants comprise proteins, and said heating step denatures said proteins causing them to precipitate.

5. A method according to any preceding claim, further characterised in that said unwanted contaminants are endonucleases, exonucleases, or proteases.

6. A method according to any preceding claim, further characterised in that the thermostable enzyme is a DNA ligase.

7. A method according to Claim 6, further characterised in that the thermostable enzyme is a DNA ligase from a thermophilic bacteria.

8. A method according to Claim 7, further characterised in that the thermostable enzyme is from a thermophilic bacterium selected from Thermus flarus, Thermus ruber, Thermus thermophilus, Bacillus stearothermophilus, Thermus aquaticus, Thermus lacteus, Thermus rubens and Methanothermus fervidus.

9. A method according to any preceding claim, further characterised in that said temperature is from 65 _{°} C to about 100°C, and preferably from 80 _{°} C to about 95 _{°} C.

10. A method for assaying for NAD-dependent thermostable nucleic acid ligase activity, characterised in comprising forming a stable covalent AMP adduct of said ligase and detecting said adduct.

11. A method according to Claim 10, further characterised in that the adenyl moiety of said adduct is labelled.

12. A method according to Claims 10 or 11, further characterised in that the adenyl moiety of said adduct is derived from NAD.

13. A method according to any of Claims 10, 11 or 12, further characterised in that said ligase is a thermophilic ligase, and said ligase activity is detected in a sample comprising undesired contaminants; and in that said method comprises heating a mixture comprising said ligase and said undesired contaminants to a temperature which denatures or inactivates said undesired contaminants but not said ligase.

14. A method of cloning nucleic acid encoding nucleic acid ligase activity, in which a library of nucleic acid segments is transformed into host cells, characterised in that said transformed host cells are screened to identify cells which express ligase activity by a method according to any of Claims 10 to 13.

15. A method according to Claim 1, further characterized in that said gene of step (a) is part of an expression vehicle comprising a promoter providing enhanced expression of said gene.

16. A method according to Claim 15, further characterized in that the expression vehicle is illustrated by the restriction map of Figure 1.

17. A method according to Claims 15 and 16, further characterized in that the ligase is NAD-dependent ligase.

## Patentansprüche (Patentansprüche für folgende(n) Vertragsstaat(en) : AT, BE, CH, LI, DE, FR, GB, IT, LU, NL, SE)

1. Ein Verfahren zur Erlangung eines von unerwünschten Verunreinigungsstoffen im wesentlichen freien thermostabilen Ligase-Enzyms, dadurch gekennzeichnet, daß es die Schritte umfaßt:
(a) Bereitstellen einer mesophilen Wirtszelle, die konstruiert wurde, um ein Gen zu exprimieren, das ein heterologes thermostabiles Ligase-Enzym codiert, wobei das Gen dadurch gekennzeichnet ist, daß es für ein Protein mit der im wesentlichen gleichen thermostabilen Ligierungsaktivität codiert wie das Protein, das durch das Gen codiert wird, das auf dem ca. 2,6 kb großen Bst XI-Bgl 11-Fragment gefunden wird, das in pGL628 (ATCC Nr. 67858) gefunden wird,
(b) Kultivieren der mesophilen Wirtszelle, um das thermostabile Enzym in einem Gemisch, das unerwünschte Verunreinigungsstoffe umfaßt, herzustellen; und
(c) Reinigen des thermostabilen Enzyms, wobei die Reinigung mindestens einen Schritt umfaßt, bei dem ein Gemisch, das die unerwünschten Verunreinigungsstoffe umfaßt, auf eine Temperatur erhitzt wird, die ausreicht, um die unerwünschten Verunreinigungsstoffe zu inaktivieren, die aber nicht ausreicht, um das thermostabile Enzym zu inaktivieren.

2. Ein Verfahren nach Anspruch 1, das dadurch weiter gekennzeichnet ist, daß nach dem Kultivierungsschritt die mesophile Wirtszelle lysiert wird, um ein Lysat herzustellen, welches das thermostabile Enzym umfaßt.

3. Ein Verfahren nach den Ansprüchen 1 oder 2, das dadurch weiter gekennzeichnet ist, daß die Reinigung das Erhitzen des unerwünschte Verunreinigungsstoffe umfassenden Gemisches umfaßt, um im wesentlichen vollständige Fällung der unerwünschten Verunreinigungsstoffe aus einer das thermostabile Enzym umfassenden flüssigen Phase zu erreichen.

4. Ein Verfahren nach Anspruch 3, das dadurch weiter gekennzeichnet ist, daß die unerwünschten Verunreinigungsstoffe Proteine umfassen und daß der Erhitzungsschritt die Proteine denaturiert, wodurch sie ausfallen.

5. Ein Verfahren nach einem vorhergehenden Anspruch, das dadurch weiter gekennzeichnet ist, daß die unerwünschten Verunreinigungsstoffe Endonucleasen, Exonucleasen oder Proteasen sind.

6. Ein Verfahren nach einem vorhergehenden Anspruch, das dadurch weiter gekennzeichnet ist, daß das thermostabile Enzym eine DNA-Ligase ist.

7. Ein Verfahren nach Anspruch 6, das dadurch weiter gekennzeichnet ist, daß das thermostabile Enzym eine DNA-Ligase aus einem thermophilen Bakterium ist.

8. Ein Verfahren nach Anspruch 7, das dadurch weiter gekennzeichnet ist, daß das thermostabile Enzym aus einem aus Thermus flarus, Thermus ruber, Thermus thermophilus, Bacillus stearothermophilus, Thermus aquaticus, Thermus lacteus, Thermus rubens und Methanothermus fervidus ausgewählten, thermophilen Bakterium stammt.

9. Ein Verfahren nach einem vorhergehenden Anspruch, das dadurch weiter gekennzeichnet ist, daß die Temperatur zwischen 65 _{°} C und ungefähr 100°C und bevorzugt zwischen 80 _{°} C und ungefähr 95 ° C liegt.

10. Ein Verfahren zur Untersuchung auf NAD-abhängige thermostabile Nucleinsäure-Ligaseaktivität, das dadurch gekennzeichnet ist, daß es die Bildung eines stabilen kovalenten AMP-Adduktes der Ligase und den Nachweis des Adduktes umfaßt.

11. Ein Verfahren nach Anspruch 10, das dadurch weiter gekennzeichnet ist, daß der Adenyl-Teil des Adduktes markiert ist.

12. Ein Verfahren nach den Ansprüchen 10 oder 11, das dadurch weiter gekennzeichnet ist, daß der Adenyl-Teil des Adduktes von NAD abgeleitet ist.

13. Ein Verfahren nach einem der Ansprüche 10, 11 oder 12, das dadurch weiter gekennzeichnet ist, daß die Ligase eine thermophile Ligase ist und daß die Ligaseaktivität in einer Probe nachgewiesen wird, die unerwünschte Verunreinigungsstoffe umfaßt, und dadurch, daß das Verfahren das Erhitzen eines die Ligase und die unerwünschten Verunreinigungsstoffe umfassenden Gemisches auf eine Temperatur umfaßt, bei der die unerwünschten Verunreinigungsstoffe, nicht aber die Ligase denaturiert oder inaktiviert werden.

14. Ein Verfahren zur Klonierung von Nucleinsäure, die Nucleinsäure-Ligaseaktivität codiert, bei dem eine Genbank von Nucleinsäuresegmenten in Wirts-Zellen transformiert wird, dadurch gekennzeichnet, daß die transformierten Wirts-zellen zur Identifizierung von Zellen durchmustert werden, die bei einem Verfahren nach einem der Ansprüche 10 bis 13 Ligaseaktivität exprimieren.

15. Ein isoliertes Nucleinsäure-Gen, das ein thermostabiles Ligase-Enzym codiert, wobei das Gen dadurch gekennzeichnet ist, daß es für ein Protein codiert mit der im wesentlichen gleichen thermostabilen Ligierungsaktivität wie das Protein, das durch das Gen codiert wird, das auf dem ca. 2,6 kb großen Bst XI-Bgl II-Fragment gefunden wird, das in pGL628 (ATCC Nr. 67858) gefunden wird.

16. Ein Gen nach Anspruch 15, das dadurch weiter gekennzeichnet ist, daß das Gen Teil eines Expressionsvehikels ist, der einen Promotor umfaßt, der eine erhöhte Expression des besagten Gens bewirkt.

17. Ein Gen nach Anspruch 16, das dadurch weiter gekennzeichnet ist, daß das Expressionsvehikel durch die Restriktionskarte der Figur 1 veranschaulicht wird.

18. Ein Gen nach Anspruch 16, das dadurch weiter gekennzeichnet wird, daß das Expressionsvehikel dasjenige ist, das unter der ATCC-Zugangs-Nr.: 67858 in E. coli hinterlegt ist.

19. Ein Gen nach einem der Ansprüche 15 bis 18, das dadurch weiter gekennzeichnet ist, daß die Ligase eine NAD-abhängige Ligase ist.

20. Eine im wesentlichen reine Nucleinsäuresequenz, die ein thermostabiles Nucleinsäure-Ligaseenzym codiert, wobei die Sequenz dadurch gekennzeichnet ist, daß sie für ein Protein codiert mit der im wesentlichen gleichen thermostabilen Ligierungsaktivität wie das Protein, das durch das Gen codiert wird, das auf dem ca. 2,6 kb großen Bst XI-Bgl 11-Fragment gefunden wird, das in pGL628 (ATCC Nr. 67858) gefunden wird.

## Patentansprüche (Patentansprüche für folgende(n) Vertragsstaat(en) : ES, GR)

1. Ein Verfahren zur Erlangung eines von unerwünschten Verunreinigungsstoffen im wesentlichen freien thermostabilen Ligase-Enzyms, dadurch gekennzeichnet, daß es die Schritte umfaßt:
(a) Bereitstellen einer mesophilen Wirtszelle, die konstruiert wurde, um ein Gen zu exprimieren, das ein heterologes thermostabiles Ligase-Enzym codiert, wobei das Gen dadurch gekennzeichnet ist, daß es für ein Protein mit der im wesentlichen gleichen thermostabilen Ligierungsaktivität codiert wie das Protein, das durch das Gen codiert wird, das auf dem ca. 2,6 kb großen Bst XI-Bgl 11-Fragment gefunden wird, das in pGL628 (ATCC Nr. 67858) gefunden wird,
(b) Kultivieren der mesophilen Wirtszelle, um das thermostabile Enzym in einem Gemisch, das unerwünschte Verunreinigungsstoffe umfaßt, herzustellen; und
(c) Reinigen des thermostabilen Enzyms, wobei die Reinigung mindestens einen Schritt umfaßt, bei dem ein Gemisch, das die unerwünschten Verunreinigungsstoffe umfaßt, auf eine Temperatur erhitzt wird, die ausreicht, um die unerwünschten Verunreinigungsstoffe zu inaktivieren, die aber nicht ausreicht, um das thermostabile Enzym zu inaktivieren.

2. Ein Verfahren nach Anspruch 1, das dadurch weiter gekennzeichnet ist, daß nach dem Kultivierungsschritt die mesophile Wirtszelle lysiert wird, um ein Lysat herzustellen, welches das thermostabile Enzym umfaßt.

3. Ein Verfahren nach den Ansprüchen 1 oder 2, das dadurch weiter gekennzeichnet ist, daß die Reinigung das Erhitzen des unerwünschte Verunreinigungsstoffe umfassenden Gemisches umfaßt, um im wesentlichen vollständige Fällung der unerwünschten Verunreinigungsstoffe aus einer das thermostabile Enzym umfassenden flüssigen Phase zu erreichen.

4. Ein Verfahren nach Anspruch 3, das dadurch weiter gekennzeichnet ist, daß die unerwünschten Verunreinigungsstoffe Proteine umfassen und daß der Erhitzungsschritt die Proteine denaturiert, wodurch sie ausfallen.

5. Ein Verfahren nach einem vorhergehenden Anspruch, das dadurch weiter gekennzeichnet ist, daß die unerwünschten Verunreinigungsstoffe Endonucleasen, Exonucleasen oder Proteasen sind.

6. Ein Verfahren nach einem vorhergehenden Anspruch, das dadurch weiter gekennzeichnet ist, daß das thermostabile Enzym eine DNA-Ligase ist.

7. Ein Verfahren nach Anspruch 6, das dadurch weiter gekennzeichnet ist, daß das thermostabile Enzym eine DNA-Ligase aus einem thermophilen Bakterium ist.

8. Ein Verfahren nach Anspruch 7, das dadurch weiter gekennzeichnet ist, daß das thermostabile Enzym aus einem aus Thermus flarus, Thermus ruber, Thermus thermophilus, Bacillus stearothermophilus, Thermus aquaticus, Thermus lacteus, Thermus rubens und Methanothermus fervidus ausgewählten, thermophilen Bakterium stammt.

9. Ein Verfahren nach einem vorhergehenden Anspruch, das dadurch weiter gekennzeichnet ist, daß die Temperatur zwischen 65 _{°} C und ungefähr 100°C und bevorzugt zwischen 80 _{°} C und ungefähr 95 _{°} C liegt.

10. Ein Verfahren zur Untersuchung auf NAD-abhängige thermostabile Nucleinsäure-Ligaseaktivität, das dadurch gekennzeichnet ist, daß es die Bildung eines stabilen kovalenten AMP-Adduktes der Ligase und den Nachweis des Adduktes umfaßt.

11. Ein Verfahren nach Anspruch 10, das dadurch weiter gekennzeichnet ist, daß der Adenyl-Teil des Adduktes markiert ist.

12. Ein Verfahren nach den Ansprüchen 10 oder 11, das dadurch weiter gekennzeichnet ist, daß der Adenyl-Teil des Adduktes von NAD abgeleitet ist.

13. Ein Verfahren nach einem der Ansprüche 10, 11 oder 12, das dadurch weiter gekennzeichnet ist, daß die Ligase eine thermophile Ligase ist und daß die Ligaseaktivität in einer Probe nachgewiesen wird, die unerwünschte Verunreinigungsstoffe umfaßt, und dadurch, daß das Verfahren das Erhitzen eines die Ligase und die unerwünschten Verunreinigungsstoffe umfassenden Gemisches auf eine Temperatur umfaßt, bei der die unerwünschten Verunreinigungsstoffe, nicht aber die Ligase denaturiert oder inaktiviert werden.

14. Ein Verfahren zur Klonierung von Nucleinsäure, die Nucleinsäure-Ligaseaktivität codiert, bei dem eine Genbank von Nucleinsäuresegmenten in Wirts-Zellen transformiert wird, dadurch gekennzeichnet, daß die transformierten Wirts-zellen zur Identifizierung von Zellen durchmustert werden, die bei einem Verfahren nach einem der Ansprüche 10 bis 13 Ligaseaktivität exprimieren.

15. Ein Verfahren nach Anspruch 1, das dadurch weiter gekennzeichnet ist, daß das Gen in Schritt (a) Teil eines Expressionsvehikels ist, das einen Promotor umfaßt, der eine gesteigerte Expression des Gens bewirkt.

16. Ein Verfahren nach Anspruch 15 , das dadurch weiter gekennzeichnet ist, daß das Expressionsvehikel durch die Restriktions-Karte der Figur 1 veranschaulicht wird.

17. Ein Verfahren nach den Ansprüchen 15 und 16, das dadurch weiter gekennzeichnet ist, daß die Ligase eine NAD-abhängige Ligase ist.

## Revendications (Revendications pour l'(les) Etat(s) contractant(s) suivant(s) : suivants : AT, BE, CH, LI, DE, FR, GB, IT, LU, NL, SE)

1. Procédé d'obtention d'une enzyme ligase thermostable pratiquement dépourvue de contaminants non désirés, caractérisé en ce qu'il comprend les étapes de :
(a) fourniture d'une cellule hôte mésophile dotée par des techniques de génie génétique de la capacité d'exprimer un gène codant pour une enzyme ligase thermostable hétérologue, le gène étant caractérisé en ce qu'il code pour une protéine ayant pratiquement la même activité de ligature thermostable que celle de la protéine codée par le gène qui se trouve dans le fragment Bst XI - Bgl Il d'environ 2,6kb contenu dans pGL628 (ATCC No. 67858)
(b) culture de la cellule hôte mésophile afin de produire l'enzyme thermostable dans un mélange comprenant des contaminants non désirés ; et
(c) purification de l'enzyme thermostable, cette purification comprenant au moins une étape dans laquelle un mélange comprenant les contaminants non désirés est chauffé à une température suffisante pour inactiver les contaminants non désirés mais insuffisante pour inactiver l'enzyme thermostable.

2. Procédé selon la revendication 1, caractérisé en outre en ce que après ladite étape de culture, les cellules hôtes mésophiles sont ly- sées pour produire un lysat comprenant ladite enzyme thermostable.

3. Procédé selon les revendications 1 ou 2, caractérisé en outre en ce que ladite purification comprend le chauffage dudit mélange comprenant lesdits contaminants non désirés afin d'effectuer une précipitation pratiquement complète desdits contaminants non désirés à partir d'une phase liquide comprenant l'enzyme thermostable.

4. Procédé selon la revendication 3, caractérisé en outre en ce que lesdits contaminants non désirés comprennent des protéines, et ladite étape de chauffage dénature lesdites protéines en provoquant leur précipitation

5. Procédé selon l'une quelconque des revendications précédentes, caractérisé en outre en ce que lesdits contaminants non désirés sont des endonucléases, des exonucléases, ou des protéases.

6. Procédé selon l'une quelconque des revendications précédentes, caractérisé en outre en ce que l'enzyme thermostable est une ligase d'ADN.

7. Procédé selon la revendication 6, caractérisé en outre en ce que l'enzyme thermostable est une ligase d'ADN provenant d'une bactérie thermophile.

8. Procédé selon la revendication 7, caractérisé en outre en ce que l'enzyme thermostable provient d'une bactérie thermophile choisie parmi Thermus flarus, Thermus ruber, Thermus thermophilus, Bacillus stearothermophilus, Thermus aquaticus, Thermus lacteus, Thermus rubens et Methanothermus fervidus.

9. Procédé selon l'une quelconque des revendications précédentes, caractérisé en outre en ce que ladite température est comprise entre 65 _{°} C et environ 100 _{°} C, et de préférence entre 80 _{°} C et environ 95 _{°} C.

10. Procédé de dosage de l'activité de ligase d'acide nucléique thermostable dépendante du NAD, caractérisé en ce qu'il comprend la formation d'un produit d'addition d'AMP covalent stable de ladite ligase et la détection dudit produit d'addition.

11. Procédé selon la revendication 6, caractérisé en outre en ce que le fragment adényle dudit produit d'addition est marqué.

12. Procédé selon les revendications 10 ou 11, caractérisé en outre en ce que le fragment adényle dudit produit d'addition est dérivé du NAD.

13. Procédé selon l'une quelconque des revendications 10, 11 ou 12, caractérisé en outre en ce que ladite ligase est une ligase thermophile, et que ladite activité de ligase est détectée dans un échantillon comprenant les contaminants non désirés ; et en ce que ledit procédé comprend le chauffage d'un mélange comprenant ladite ligase et lesdits contaminants non désirés à une température qui dénature ou inactive lesdits contaminants non désirés mais pas ladite ligase.

14. Procédé de clonage d'acide nucléique codant pour une activité de ligase d'acide nucléique, dans lequel on transforme des cellules hôtes par des segments d'acide nucléique d'une banque, caractérisé en ce que lesdites cellules hôtes transformées sont criblées de façon à identifier les cellules qui expriment l'activité de ligase par un procédé conforme à l'une quelconque des revendications 10 à 13.

15. Gène d'acide nucléique isolé, qui code pour une enzyme ligase thermostable, le gène étant caractérisé en ce qu'il code pour une protéine ayant pratiquement la même activité de ligature thermostable que celle de la protéine codée par le gène qui se trouve dans le fragment Bst XI - Bgl Il d'environ 2,6kb situé dans pGL628 (ATCC N° . 67 858).

16. Gène selon la revendication 15, caractérisé en outre en ce que ledit gène fait partie d'un vecteur d'expression comprenant un promoteur permettant d'obtenir une expression activée dudit gène.

17. Gène selon la revendication 16, caractérisé en outre en ce que le vecteur d'expression est illustré par la carte de restriction de la figure 1.

18. Gène selon la revendication 16, caractérisé en outre en ce que le vecteur d'expression est celui déposé sous le numéro d'accession ATCC 67858 dans E. coli.

19. Gène selon l'une quelconque des revendications 15 à 18, caractérisé en outre en ce que la ligase est une ligase dépendante du NAD.

20. Séquence d'acide nucléique pratiquement pure qui code pour une enzyme ligase d'acide nucléique thermostable, la séquence étant caractérisée en ce qu'elle code pour une protéine ayant pratiquement la même activité de ligature thermostable que celle de la protéine codée par le gène qui se trouve dans le fragment Bst XI - Bgl Il d'environ 2,6kb situé dans pGL628 (ATCC N °. 67858).

## Revendications (Revendications pour l'(les) Etat(s) contractant(s) suivant(s) : suivants : ES, GR)

1. Procédé d'obtention d'une enzyme ligase thermostable pratiquement dépourvue de contaminants non désirés, caractérisé en ce qu'il comprend les étapes de :
(a) fourniture d'une cellule hôte mésophile dotée par des techniques de génie génétique de la capacité d'exprimer un gène codant pour une enzyme ligase thermostable hétérologue, le gène étant caractérisé en ce qu'il code pour une protéine ayant pratiquement la même activité de ligature thermostable que celle de la protéine codée par le gène qui se trouve dans le fragment Bst XI - Bgl Il d'environ 2,6kb contenu dans pGL628 (ATCC No. 67858)
(b) culture de la cellule hôte mésophile afin de produire l'enzyme thermostable dans un mélange comprenant des contaminants non désirés ; et
(c) purification de l'enzyme thermostable, cette purification comprenant au moins une étape dans laquelle un mélange comprenant les contaminants non désirés est chauffé à une température suffisante pour inactiver les contaminants non désirés mais insuffisante pour inactiver l'enzyme thermostable.

2. Procédé selon la revendication 1, caractérisé en outre en ce que après ladite étape de culture, les cellules hôtes mésophiles sont ly- sées pour produire un lysat comprenant ladite enzyme thermostable.

3. Procédé selon les revendications 1 ou 2, caractérisé en outre en ce que ladite purification comprend le chauffage dudit mélange comprenant lesdits contaminants non désirés afin d'effectuer une précipitation pratiquement complète desdits contaminants non désirés à partir d'une phase liquide comprenant l'enzyme thermostable.

4. Procédé selon la revendication 3, caractérisé en outre en ce que lesdits contaminants non désirés comprennent des protéines, et ladite étape de chauffage dénature lesdites protéines en provoquant leur précipitation

5. Procédé selon l'une quelconque des revendications précédentes, caractérisé en outre en ce que lesdits contaminants non désirés sont des endonucléases, des exonucléases, ou des protéases.

6. Procédé selon l'une quelconque des revendications précédentes, caractérisé en outre en ce que l'enzyme thermostable est une ligase d'ADN.

7. Procédé selon la revendication 6, caractérisé en outre en ce que l'enzyme thermostable est une ligase d'ADN provenant d'une bactérie thermophile.

8. Procédé selon la revendication 7, caractérisé en outre en ce que l'enzyme thermostable provient d'une bactérie thermophile choisie parmi Thermus flarus, Thermus ruber, Thermus thermophilus, Bacillus stearothermophilus, Thermus aquaticus, Thermus lacteus, Thermus rubens et Methanothermus fervidus.

9. Procédé selon l'une quelconque des revendications précédentes, caractérisé en outre en ce que ladite température est comprise entre 65 _{°} C et environ 100° C, et de préférence entre 80 _{°} C et environ 95 _{°} C.

10. Procédé de dosage de l'activité de ligase d'acide nucléique thermostable dépendante du NAD, caractérisé en ce qu'il comprend la formation d'un produit d'addition d'AMP covalent stable de ladite ligase et la détection dudit produit d'addition.

11. Procédé selon la revendication 6, caractérisé en outre en ce que le fragment adényle dudit produit d'addition est marqué.

12. Procédé selon les revendications 10 ou 11, caractérisé en outre en ce que le fragment adényle dudit produit d'addition est dérivé du NAD.

13. Procédé selon l'une quelconque des revendications 10, 11 ou 12, caractérisé en outre en ce que ladite ligase est une ligase thermophile, et que ladite activité de ligase est détectée dans un échantillon comprenant les contaminants non désirés ; et en ce que ledit procédé comprend le chauffage d'un mélange comprenant ladite ligase et lesdits contaminants non désirés à une température qui dénature ou inactive lesdits contaminants non désirés mais pas ladite ligase.

14. Procédé de clonage d'acide nucléique codant pour une activité de ligase d'acide nucléique, dans lequel on transforme des cellules hôtes par des segments d'acide nucléique d'une banque, caractérisé en ce que lesdites cellules hôtes transformées sont criblées de façon à identifier les cellules qui expriment l'activité de ligase par un procédé conforme à l'une quelconque des revendications 10 à 13.

15. Procédé selon la revendication 1, caractérisé en outre en ce que ledit gène de l'étape (a) fait partie d'un vecteur d'expression comprenant un promoteur permettant d'obtenir une expression activée dudit gène.

16. Procédé selon la revendication 15, caractérisé en outre en ce que le vecteur d'expression est illustré par la carte de restriction de la figure 1.

17. Procédé selon les revendications 15 et 16, caractérisé en outre en ce que la ligase est une ligase dépendante du NAD.
